(19) **Europäisches Patentamt / European Patent Office / Office européen des brevets**

(11) **EP 3 359 035 B1**

(12) # EUROPEAN PATENT SPECIFICATION

(45) Date of publication and mention of the grant of the patent:
**29.12.2021 Bulletin 2021/52**

(51) Int Cl.:
*A61B 5/08* (2006.01)  *A61B 5/091* (2006.01)
*A61B 5/087* (2006.01)  *A61B 5/097* (2006.01)
*A61M 16/10* (2006.01)

(21) Application number: **16775595.8**

(22) Date of filing: **23.09.2016**

(86) International application number:
**PCT/EP2016/072622**

(87) International publication number:
**WO 2017/060097 (13.04.2017 Gazette 2017/15)**

(54) **DEVICE AND SYSTEM FOR DETERMINING A RESPIRATORY FEATURE OF A SUBJECT BASED ON A BREATHING GAS**

VORRICHTUNG UND SYSTEM ZUR BESTIMMUNG EINES RESPIRATORISCHEN MERKMALS EINER PERSON AUF DER BASIS EINES ATEMGASES

DISPOSITIF ET SYSTÈME PERMETTANT DE DÉTERMINER UNE CARACTÉRISTIQUE RESPIRATOIRE D'UN SUJET SUR LA BASE D'UN GAZ RESPIRATOIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.10.2015 EP 15188799**

(43) Date of publication of application:
**15.08.2018 Bulletin 2018/33**

(73) Proprietor: **Koninklijke Philips N.V.**
**5656 AG Eindhoven (NL)**

(72) Inventors:
• **KARAKAYA, Koray**
**5656 AE Eindhoven (NL)**
• **VAN LIESHOUT, Ron Martinus Laurentius**
**5656 AE Eindhoven (NL)**
• **KUENEN, Maarten Petrus Joseph**
**5656 AE Eindhoven (NL)**
• **DE SAMBER, Marc Andre**
**5656 AE Eindhoven (NL)**

(74) Representative: **Philips Intellectual Property & Standards**
**High Tech Campus 52**
**5656 AG Eindhoven (NL)**

(56) References cited:
WO-A1-2014/076250    US-A- 4 370 986
US-A- 4 517 987      US-A1- 2005 066 968

US-A1- 2008 017 198

• William C Hinds: "Respiratory Deposition" In: "Aerosol Technology: Properties, Behavior, and Measurement of Airborne Particles, 2nd Edition", 28 February 1999 (1999-02-28), Wiley, XP055258617, ISBN: 978-0-471-19410-1 pages 232-259, figure 11.3 page 244 - page 245
• G. INVERNIZZI ET AL: "Real-time measurement of particulate matter deposition in the lung", BIOMARKERS, vol. 11, no. 3, 1 January 2006 (2006-01-01), pages 221-232, XP055258620, GB ISSN: 1354-750X, DOI: 10.1080/13547500600648523
• Kuo-Hsi Cheng ET AL: "Calculation of Total Deposition Fraction of Ultrafine Aerosols in Human Extrathoracic and Intrathoracic Regions", AEROSOL SCIENCE AND TECHNOLOGY., vol. 22, no. 2, 1 January 1995 (1995-01-01), pages 194-201, XP055720926, US ISSN: 0278-6826, DOI: 10.1080/02786829509508887

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**EP 3 359 035 B1**

**(Cont. next page)**

- B Lehnigk ET AL: "Aerosol-derived airway morphometry (ADAM) in patients with lung emphysema diagnosed by computed tomography--reproducibility, diagnostic information and modelling", European Journal of Medical Research, 26 February 2007 (2007-02-26), pages 74-83, XP055720933, Retrieved from the Internet: URL:https://daignet.de/site-content/die-da ig/fachorgan/archiv/2007-1/ejomr-2007_2-pd fs/S.74_Lehnigk.pdf [retrieved on 2020-08-07]

- P Brand ET AL: "Can Aerosol-Derived Airway Morphometry Detect Early, Asymptomatical Lung Emphysema?", JOURNAL OF AEROSOL MEDICINE, 1 January 2003 (2003-01-01), pages 143-151, XP055720930, Retrieved from the Internet: URL:https://www.liebertpub.com/doi/pdf/10. 1089/089426803321919906 [retrieved on 2020-08-07]

## Description

FIELD OF THE INVENTION

The present invention relates to a device and system for determining a respiratory feature of a subject based on a breathing gas, in particular an inhaled or exhaled gas. It finds application in monitoring of lung status and functionality, in particular for management or treatment of a broad class of respiratory diseases, such as COPD, asthma, lung emphysema, as well as non-respiratory diseases, such as heart failure, that show respiratory symptoms. Further, the present invention finds application in products related to air quality such as air purification products and in respiratory support products.

BACKGROUND OF THE INVENTION

Respiratory diseases have become more and more frequent in recent years. In the case of respiratory diseases, such as chronic obstructive pulmonary disease (COPD), lung emphysema or asthma, the severity of symptoms depends strongly on environmental conditions and the physical condition of the patient. Because these factors influence the instantaneous respiratory features such as lung capacity of the patient, the respiratory features are important clinical parameters in the management of these diseases.

Considering the relationship between respiratory features, in particular lung capacity, and the severity of COPD symptoms, it is highly desirable to know the instantaneous level of the respiratory features, in particular those relating to a specific status of the respiratory tract, in order to improve and personalize COPD management.

Numerous technologies for assessment of pulmonary function and, in particular, lung capacity, are available. These approaches are, however, based on dedicated clinical tests that have significant drawbacks. For instance, the known approaches are highly advanced clinical tests that require dedicated, costly equipment that are typically only available at highly specialized medical centers. More simple measurement principles require the presence of a skilled doctor to oversee correct implementation of the test. In addition, these tests are based on capacity/exercise tests that often cause pains to the patient.

Due to various reasons, the approaches for determining pulmonary function are, in practice exclusively available in clinical settings, generally under expert supervision. However, as symptoms in patients with respiratory diseases may vary significantly on a very short term, there is a need for ambulatory pulmonary function tests which are reliable and capable of supporting short-term management of respiratory diseases, such as COPD, asthma, and lung emphysema.

WO 2014/076250A1 describes a device for pulmonary function measurement in a subject. The device comprises a means for providing an aerosol comprising particles to be inhaled by the subject, and a means for analysis of a portion of air exhaled by the subject, wherein said particles are hydrophobic and are within the range of 20 - 200 nm, preferably 50 - 100 nm, and wherein said portion is exhaled after exhalation of a volume corresponding to dead space.

US 2005/066968A1 describes that for controlled delivery of medicament to the respiratory system of a patient, a measured quantity of a medicament, i.e., a bolus is introduced into the inspiratory flow stream of a patient, via a breathing tube, and inhaled by the action of the patient's breathing cycle of the course of a breathing cycle of the patient, numerous properties or characteristics of the medicament, i.e., the bolus, the inspiratory flow, like properties or characteristics of the expiratory flow, and/or other useful information are derived. The derived information is employed to control subsequent delivery of boli to the patient, including the delivery of each bolus as a function of the commencement or progression of the breathing cycle, as input for calculations or determinations which are useful in analyzing the effectiveness of delivery of the medicament to the patient, patient compliance, direction of flow through the breathing tube, and other uses.

US2005/0045175A1 proposes a method for measuring lung ventilation by calculating a time constant based on a volume of inhalation converted from pressure data. The proposed method enables to monitor or treat chronic respiratory diseases irrespective of the health condition of the patient. However, the proposed method is limited in the measurement accuracy and the capability of accessing the respiratory tract, so that the lung ventilation measurement is less reliable.

SUMMARY OF THE INVENTION

It is an object of the present invention to provide a device and system for determining a respiratory feature of a subject which enable to increase the measurement accuracy and the capability of accessing the respiratory tract as well as easier usage, in particular simplified self-assessment or home-use.

In a first aspect of the present invention a device for determining a respiratory feature of a subject according to claim 1 is provided.

In a further aspect of the present invention a system for determining a respiratory feature of a subject according to claim 12 is provided.

In a further aspect not according to the present invention a method for determining a respiratory feature of a subject, in particular a living being, based on a breathing gas generated by the subject when inhaling and/or exhaling, is provided that comprises the steps of detecting an aerosol contained in a breathing gas, measuring a deposition fraction for the detected aerosol, wherein the deposition fraction indicates the fraction of aerosol deposited inside the subject over the total amount of the inhaled aerosol, and determining the respiratory feature by relating the measured deposition fraction to a plurality of predetermined deposition fractions each corresponding to a different airway geometry of a respiratory tract.

In yet further aspects not according to the present invention, there are provided a computer program which comprises program code means for causing a computer to perform the steps of the method disclosed herein when the computer program is carried out on a computer as well as non-transitory computer-readable recording medium that stores therein a computer program product, which, when executed by a device, causes the method disclosed herein to be performed.

Preferred embodiments of the invention are defined in the dependent claims. It shall be understood that the claimed system has similar and/or identical preferred embodiments as the claimed device and as defined in the dependent claims.

The present invention enables a simplified approach to assess the respiratory tract of a subject, such as a patient. The term "breathing gas" is to be understood generally and is not restricted to substances of gaseous form. In particular, the breathing gas may contain an aerosol. When the patient generates a breathing gas, e.g. by inhaling through the device and exhaling into the device within an environment where aerosols are present, the generated breathing gas containing an aerosol reaches the aerosol detection unit, e.g. an aerosol sensor, so that the aerosol is detected. During inhalation, the gas from the environment is breathed/inhaled into the respiratory tract and at this moment it becomes a breathing gas. During exhalation, the breathing gas within the respiratory tract is breathed/exhaled into the environment. A breathing gas is hence the result of an inhale (breath in) and exhale (breath out) action, during which the aerosol/particles may change. Such a change may provide an indication of the lung status.

The term "aerosol" refers to a mixture or colloid of substances such as solid particles or liquid droplets, in a gas such as the air and the atmosphere. In particular, aerosols refer to a variety of different particles such as smog, automobile exhaust particulates, tobacco smoke, virus, bacteria, fog, pollen, fungal spores, dusts, allergens, etc. Aerosols can also be referred under different size range classifications such as PM10, PM2.5, PM1, and UFP (ultrafine particle). The device can be integrated into a system which further comprises a gas inlet/outlet, e.g. a mouthpiece, connected to the aerosol detection unit. Alternatively, the device itself may comprise the gas inlet/outlet or mouthpiece.

Upon detection of the aerosol, the deposition fraction is measured for the detected aerosol by the deposition fraction unit. The deposition fraction is generally defined as the amount of material inhaled by a subject and deposited in one or more tissues or parts of the respiratory tract of the subject divided by the total amount of the inhaled material.

The deposition fraction may be obtained based on the volume, mass, pressure and/or concentration/density of the inhaled and/or exhaled aerosol, such as during one or more breath cycles each comprising an inhalation phase followed by an exhalation phase. By measuring the total amount of inhaled aerosol and the amount of subsequently exhaled aerosol, the fraction of aerosol deposited in the respiratory tract, e.g. lungs, can be calculated. If one of these two quantities is already known, e.g. from earlier measurements, only the other quantity needs to be measured in order to calculate the deposition fraction for the detected aerosol.

In order to determine a respiratory feature of the subject, the respiratory feature determination unit is able to relate the measured deposition fraction to a plurality of predetermined deposition fractions. Each of the predetermined deposition fractions is characteristic for a specific airway geometry of a respiratory tract. The respiratory tract of a living being, in particular human, is the ensemble of respiratory tissues/organs that form the airways of the entire respiratory system of the living being. In particular, such tissues/organs include nose, mouth, pharynx, larynx, trachea, lungs, bronchi, bronchial tree, tracheobronchial airways, pulmonary airways, upper airways and lower airways.

The different airways are characterized by their different geometries, wherein for each specific airway geometry the deposition fraction can be predetermined, e.g. by the deposition fraction measurement unit or by an external unit, in particular for a human or animal or by simulation. Preferably, the deposition fractions have been predetermined for the subject whose respiratory feature is to be determined. In this way, the relating of the measured deposition fraction to the predetermined deposition fractions is facilitated so that the determination of respiratory feature is more reliable. In particular, anatomical variations in subjects can be counter-acted/taken into account. Alternatively or additionally, the predetermined deposition fractions can be retrieved by the respiratory feature determination unit, e.g. from a server, data carrier/storage, or via user input.

The respiratory feature determination unit is preferably configured to perform at least one of the following processes: comparing the measured deposition fraction with a plurality of predetermined deposition fractions, e.g. concerning the form and/or value of the measured curve; computing a correlation function between the measured deposition fraction and a plurality of predetermined deposition fractions; representing the measured deposition fraction as a function of the plurality of predetermined deposition fractions. Still further ways of relating the measured deposition fraction to the predetermined deposition fractions can be used.

The respiratory feature may be, without being limited to, one of the following examples: lung capacity (LC), lung depth (LD), total lung capacity (TLC), inspiratory capacity (IC), tidal volume ($V_T$), functional residual capacity (FRC), expiratory residual volume (ERV), vital capacity (VC), residual volume (RV).

Advantageously, the present invention enables to determine respiratory features based on a breathing gas. In particular, the respiratory features can be determined based on poly-disperse aerosols, e.g. aerosols that contain particles of different sizes. This is because deposition fraction is measured for the detected aerosol and such a measurement is not restricted to a specific aerosol composition, in particular a specific particle size. In particular, the size range of the deposited aerosols may serve as an indicator of the lung capacity. Therefore, using poly-disperse aerosols instead of mono-disperse aerosols is advantageous while enabling to work with ambient aerosols. This means that the determination is not only possible for one specific aerosol composition or size distribution, but any, in particular uncontrolled composition or ambient aerosols. Hence, the determination of respiratory features is not affected by changes of aerosol composition, so that continuous assessment of the respiratory tract is facilitated.

Further, this enables respiratory feature determination even under uncontrolled breathing conditions of the patient, e.g. not well-defined breath volumes or breathing gas velocities, etc. The deposition fraction can be measured for the detected aerosol based on the accessible sections of the respiratory tract, in particular the lung, so that the respiratory feature, e.g. lung capacity, can be determined reliably. This is particularly advantageous compared to known approaches which require a mono-disperse aerosol which has a predefined composition, where continuous assessment or assessment under uncontrolled conditions is impossible or at least less reliable.

In addition, by taking the different airway geometries of the respiratory tract into account, the present invention is also advantageous over approaches based purely on breath volume measurement. In particular, the breath volume depends both on the actual size of the lungs and on the physical activity level of the body at the time of lung capacity assessment. In this way, only a general assessment can be provided that may not take specific sections of the respiratory tract that are not (easily) accessible into account, especially when the physical activity of the patient is low.

In contrast, such "difficult" sections can be addressed by the present invention, e.g. by relating the measured deposition fraction to the deposition fractions predetermined for the "difficult" sections. Therefore, the respiratory feature determined in this way conveys an improved indicator for the respiratory tract capacity, in particular the lung capacity.

According to the invention, the deposition fraction measurement unit is configured to measure a particle concentration for a plurality of particle sizes of the detected aerosol. The deposition fraction measured in this way is a function of the aerosol/particle size, in particular the diameter of the particles contained in the detected aerosol. Monitoring a cluster of various particle sizes contained in the detected aerosol can yield multiple values of deposition fraction each corresponding to a specific particle size, so that more detailed data about the lung capacity are obtainable. Advantageously, the accuracy of the measurement is increased.

Preferably, the deposition fraction measurement unit is configured to derive a particle size distribution of the detected aerosol, i.e. a distribution of amount, volume or concentration of the detected aerosol particles depending on the particle size. For instance, this may be done for one or multiple breath cycles. From the relation or ratio between the size distribution of the inhaled aerosol and that of the exhaled aerosol, clinically relevant features related to lung capacity can be measured. In particular, this approach allows measuring of the depth of breathing.

Moreover, the ability to measure the aerosol size distribution in any volume of breathing gas allows high flexibility in defining specific measurement conditions to apply the present invention. Alternatively or additionally, the deposition fraction measurement unit is configured to derive a total deposition fraction being the sum of deposition fractions measured at the plurality of particle sizes of the detected aerosol.

In another preferable embodiment, the respiratory feature determination unit is configured to represent the measured deposition fraction as a function of the plurality of predetermined deposition fractions. In this way, the deposition fraction of the detected aerosol is regarded as a function of the deposition fractions that have previously been determined for different parts of the respiratory tract of the subject. Advantageously, one can take the contributions of different airway geometries, e.g. parts/sections, of the respiratory tract to the measured deposition fraction into account, so that the respiratory feature, e.g. lung capacity, is determined with higher accuracy. Alternatively or additionally, the deposition fraction is measured at a plurality of particle sizes/diameters of the detected aerosol.

Preferably, the function comprises summation of the plurality of predetermined deposition fractions or their subfunctions, wherein the predetermined deposition fractions or their subfunctions are each multiplied by a corresponding one of a plurality of weighting factors. Hence, the measured deposition fraction is represented as summation of predetermined deposition fractions each multiplied with a weighting factor, or alternatively as summation of subfunctions (e.g. polynomials, exponential functions and/or logarithmic functions) of the predetermined deposition fractions, wherein each subfunction is multiplied by a corresponding weighting factor. Advantageously, using summation simplifies the determination of the respiratory feature compared to using more complex functions. Alternatively or additionally, the summation is a weighted average of the plurality of predetermined deposition fractions or their subfunctions. In particular, using subfunctions enables to perform more complex lung assessments including e.g. multi-symptom effects.

Further preferably, the respiratory feature determination unit is configured to derive the respiratory feature from the plurality of weighting factors. If a weighting factor for the predetermined deposition fraction which corresponds to a specific part/section of the respiratory tract is relatively high, the contribution of this part/section to the measured deposition fraction of the detected aerosol is relatively high. By computing the weighting factors, the contributions of different parts/sections of the respiratory tract to the measured deposition fraction can be directly derived each from the corresponding weighting factor. Since the contributions are indicative of the level/state of a respiratory feature, the latter, e.g. lung capacity or depth of breath, can be easily determined based on the relative contributions of the parts/sections.

In another preferable embodiment, the weighting factor corresponding to one or more specific airway geometries is compared with a predefined threshold characterizing a respiratory feature. Alternatively or additionally, the ratio between at least two weighting factors each corresponding to a specific airway geometry is determined, and preferably compared with a predefined ratio characteristic for a respiratory feature. In another embodiment, the weighting factors determined as described above can be used as indicator of additional characteristics of the lung functionality/status. Changes of the determined weighting factors over time can be used to determine e.g. onset of other clinical effects (e.g. infections).

In another preferable embodiment, the device further comprises a monitoring unit for monitoring an air composition. The monitoring unit may comprise an air quality detection device, a detector for measuring the size and/or the concentration of particulate matter (PM), and/or for a specific size range, e.g. PM2.5 whose diameter is up to 2.5 micrometre ($\mu$m), or PM10 whose diameter is up to 10 micrometre ($\mu$m). Alternatively, the monitoring of air composition can be performed by the aerosol detection unit and/or the deposition fraction measurement unit. Advantageously, the monitored air composition provides additional information about the environment in which the subject is located.

In another preferable embodiment, the device is a portable or wearable device, and/or connectable to an external monitoring unit for monitoring an air composition. A portable/wearable device is advantageous in providing high mobility when using the device. In particular, the user can perform self-assessment of lung capacity wherever he is located and at any given time. The external monitoring unit may be an (smart) air cleaner, air conditioner, an air purification product or the like.

Preferably, the connection between the device and the external monitoring unit is a wireless link, e.g. based on infrared, Wi-Fi and/or Bluetooth. Additionally or alternatively, the device functions as a "master" capable of receiving data from and/or sending commands to the external monitoring unit which functions as a "slave". Further, the external monitoring unit may be positioned closely distanced from the device, preferably in the same room as the device for indoor applications. Preferably, the external monitoring unit is for measuring air with similar composition to that at the place of the device. If one of the external monitoring unit and the device is close to an aerosol source or in close proximity of an air purifier, there may be significant differences in measured air composition and/or aerosol concentration for the external monitoring unit and the device.

In another preferable embodiment, the device further comprises a guiding unit for defining a time point and/or location for determining the respiratory feature. The time point and/or location can be defined based on monitoring of air composition and/or measurement of size distribution of aerosol particles. For instance, a place in the house with a 'good' composition (e.g. ambient aerosol composition) suitable for conducting lung assessment can be found by e.g. air composition monitoring.

In particular, the monitoring (e.g. instantaneous or continuous monitoring by the integrated or external monitoring unit) may be done in pre-set timing intervals or activated by user input. In this way, an optimal time point and/or location for assessment of respiratory tract, e.g. lung capacity is defined. Additionally or alternatively, the guiding unit comprises a user interface capable of signalling the user such as using display, audio/speech output, LED, vibration.

BRIEF DESCRIPTION OF THE DRAWINGS

These and other aspects of the invention will be apparent from and elucidated with reference to the embodiment(s) described hereinafter. In the following drawings

Fig. 1 shows schematically a diagram of a plurality of respiratory features related to lungs;
Fig. 2 shows schematically a device for determining a respiratory feature according to an embodiment;
Fig. 3 shows schematically a device for determining a respiratory feature according to another embodiment;
Fig. 4A shows schematically a device according to a further embodiment operated to monitor air composition;

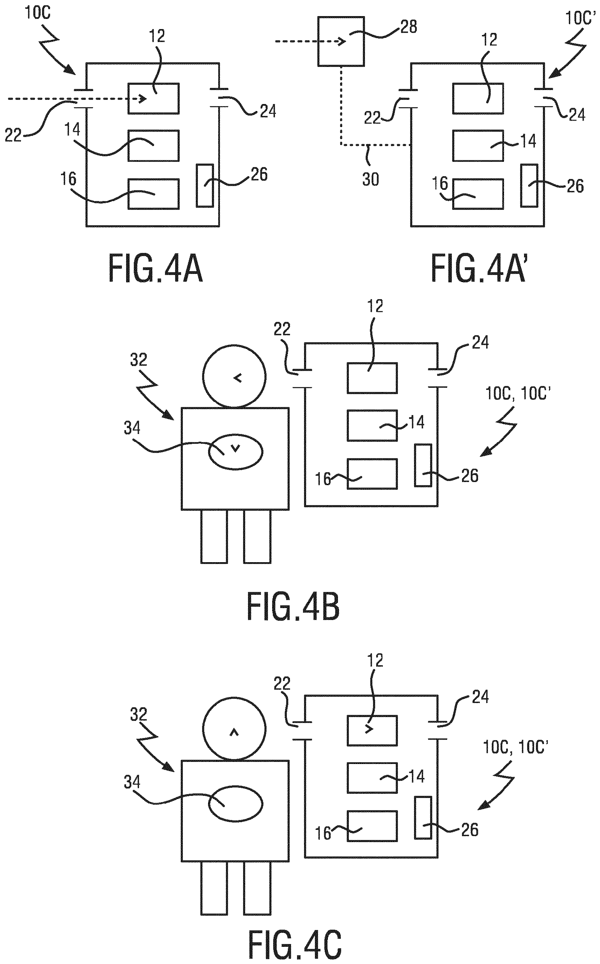

Fig. 4A' shows schematically a device according to a further embodiment controlling an external monitoring unit for monitoring air composition;
Fig. 4B shows schematically the device shown in Fig. 4A, 4A' operated during an inhalation phase of a subject;
Fig. 4C shows schematically the device shown in Fig. 4A, 4A' operated during an exhalation phase of the subject;

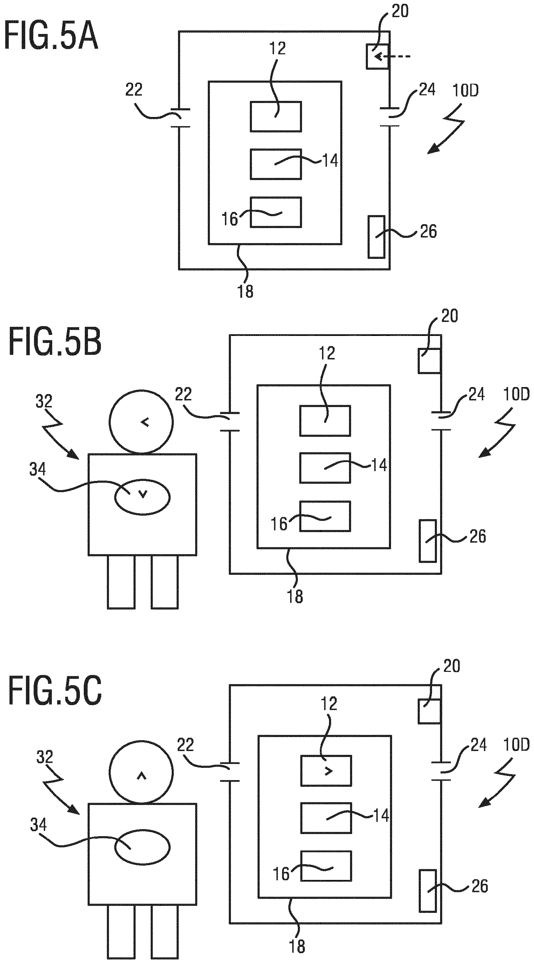

Fig. 5A shows schematically a device according to a further embodiment operated to monitor air composition;
Fig. 5B shows schematically the device shown in Fig. 5A operated during an inhalation phase of a subject;

Fig. 5C shows schematically the device shown in Fig. 5A operated during an exhalation phase of the subject;

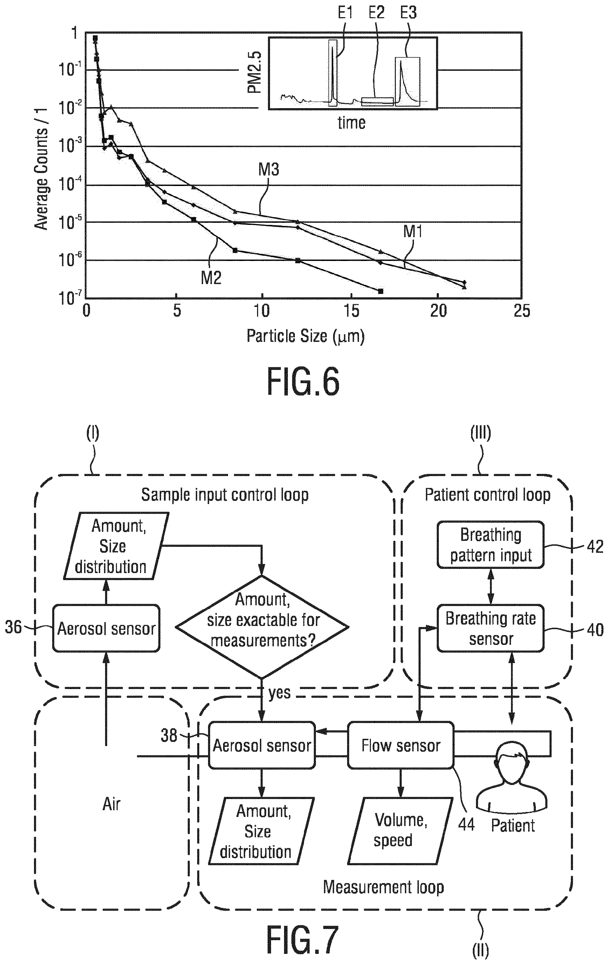

Fig. 6 shows schematically an example of deposition fraction plotted against aerosol size distribution corresponding to three different indoor events indicated in the inset;

Fig. 7 shows schematically a procedure flow scheme for lung capacity determination; and

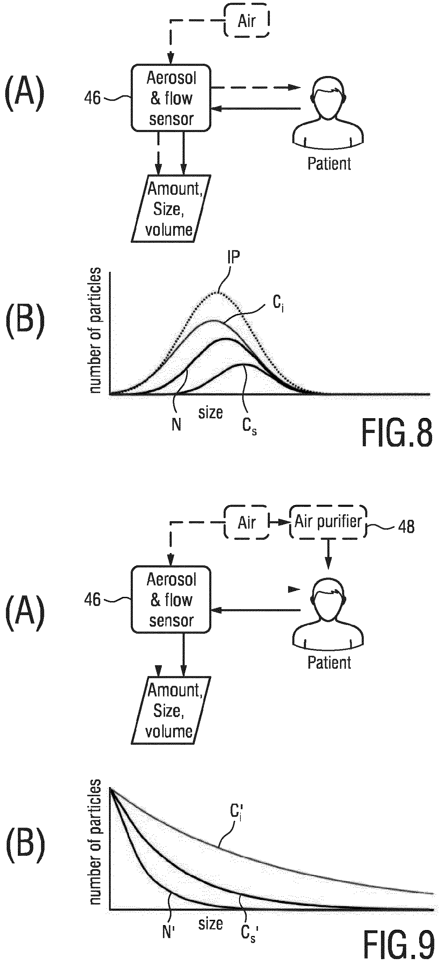

Figs. 8-9 show schematically another procedure flow scheme for lung capacity determination.

DETAILED DESCRIPTION OF THE INVENTION

Based on the general concept of measuring the deposition fraction, in particular the size distribution of inhaled and exhaled aerosol, different approaches are possible for assessment of respiratory features such as those related to lung capacity. Such assessments provide an important indicator of respiratory diseases status such as in COPD patients. In this regard, embodiments are proposed that are suitable for home-use and/or for use in combination with existing products in the fields of air quality as well as respiratory support products.

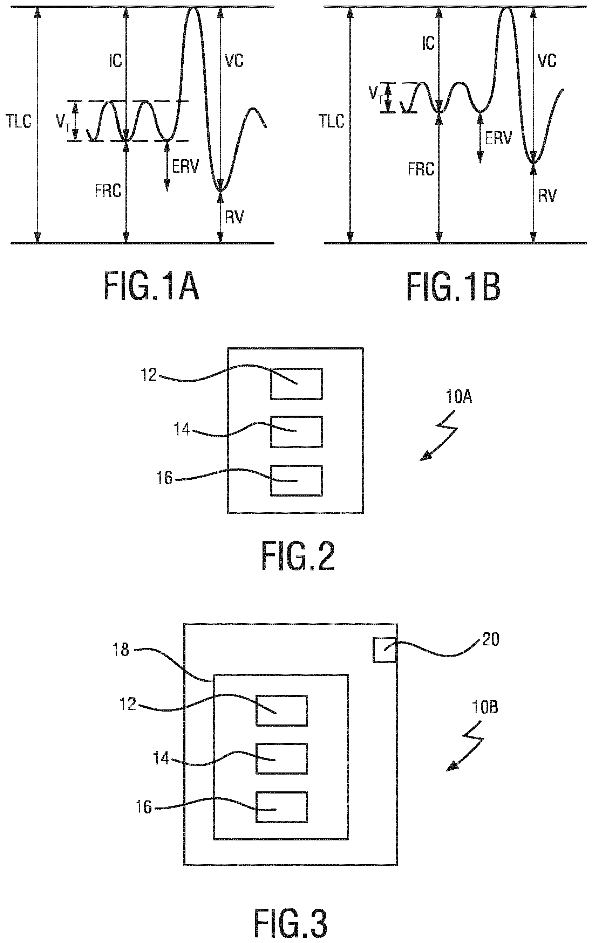

Fig. 1A-B shows schematically a diagram of a plurality of respiratory features related to lungs, including lung capacity (LC), lung depth (LD), total lung capacity (TLC), inspiratory capacity (IC), tidal volume ($V_T$), functional residual capacity (FRC), expiratory residual volume (ERV), vital capacity (VC), residual volume (RV), forced respiratory volume (FRV). The $V_T$ is the volume of breathing gas inhaled and exhaled during normal breathing (e.g. at rest). The IC is the volume that a person can maximally inhale after a normal exhalation. The VC is the volume that a person can maximally inhale and exhale.

In Fig. 1A, the lung volumes and capacities in normal/healthy conditions are shown. In Fig. 1B, the (hyper-inflated) lung volumes and capacities in conditions like COPD are shown, where changes with respect to the normal conditions are visible. Notably, COPD patients typically have an increased ERV inside the lungs, both during normal breathing (characterized by an increased FRV) and during maximum expiration (characterized by an increased RV). As a result, COPD patients have a lower lung capacity as evidenced by a decrease in the parameters IC, VC, and $V_T$. As shown in Fig. 1B, COPD patients typically have more shallow breathing (i.e. indicated by smaller $V_T$) than healthy persons. Thus, it is desirable to determine the depth of breath of a patient to gain information about his lung capacity.

Since the lung capacity and the severity of COPD symptoms are correlated, it is highly desirable to know the instantaneous lung capacity in order to improve and personalize COPD management. Current approaches for measurement of lung capacity and, more generally, pulmonary function, are, however, based on dedicated clinical tests that have significant drawbacks.

A known test in the management of COPD used for estimation of the patient's vital capacity is the Forced Expiratory Volume (FEV) test. In this test, typically performed using spirometry, the maximum volume of breathing gas exhaled by the patient is measured by a doctor. This test is, however, rather painful for COPD patients to perform due to their difficulty to exhale properly. Moreover, the application of FEV is mostly limited to clinical settings in practice.

Another typical test performed in COPD patients is the "six-minute walk test", as the distance walked and the oxygen saturation measured during and after exercises of the patient are indicative of the pulmonary status. However, like the FEV test, this test is also not suitable for routine monitoring solutions since COPD patients often experience intensive pain/uncomfortableness during the test.

Pulmonary function can also be assessed by arterial blood gas analysis, in which the oxygen and carbon dioxide concentrations in arterial blood are measured. This analysis, however, requires arterial blood sampling, hence is rather obtrusive.

Dedicated lung volume assessments are possible using techniques such as helium dilution. In these techniques, the patient inhales a breathing gas with a different gas composition (e.g. normal air with added helium, or pure oxygen) than atmospheric air. By analysis of the gas composition of the exhaled gas, pulmonary volumes, including the functional dead space (i.e. the volume of air which is inhaled that does not take part in the gas exchange), can be estimated. These techniques, however, require the availability of dedicated air compositions and are, therefore, limited to clinical settings.

A further approach involves capnography, which is used to measure the carbon dioxide concentration/partial pressure in air. This technique is commonly used in patients that are mechanically ventilated (e.g. during surgery). This method not only measures pulmonary function but also cardiac output and is a useful marker for metabolic activity.

Still further technologies are based on aerosols instead of gases. These technologies are aerosol bolus dispersion (ABD) and aerosol-derived airway morphometry (ADAM). In such methods, the patient inhales a well-defined bolus of mono-disperse aerosol containing particles of a specific size (a common exemplary size of the particles contained in the aerosol is 1 μm). Since the size of aerosol particles is a main determinant of their deposition depth in the airways of the lungs, the particle concentration in the exhaled aerosol can be interpreted in terms of intrapulmonary airspace dimensions (or lung capacity).

Fig. 2 shows schematically a device 10A for determining a respiratory feature according to an embodiment.

The device 10A comprises an aerosol detection unit 12 for detecting an aerosol content in a breathing gas generated by a subject, a deposition fraction measurement unit 14 for measuring a deposition fraction (DF) for the detected aerosol and a respiratory feature determination unit 16 for determining a respiratory feature by relating the measured deposition fractions to a plurality of predetermined deposition fractions each corresponding to a different airway geometry of a respiratory tract.

Fig. 3 shows schematically a device 10B for determining a respiratory feature according to another embodiment.

The device 10B is similar to the device 10A shown in Fig. 2, except that the device 10B comprises a monitoring unit 20 for monitoring an air composition in addition. The aerosol detection unit 12, the deposition fraction measurement unit 14 and the respiratory feature examination unit 16 form together an assessment section 18.

Fig. 4A shows schematically a device 10C according to a further embodiment. The device 10C is similar to the device 10A shown in Fig. 2, except that the device 10C further comprises a first gas opening 22 and a second gas opening 24. Both gas openings 22, 24 are coupled to the aerosol detection unit 12 to form a gas channel. The gas openings 22, 24 are preferably a mouthpiece or the like which enables airtight connection to the patient's respiratory organ such as mouth and/or nose. Further, the device 10C comprises a guiding unit 26 for setting a time point and/or location for assessment of the respiratory tract, e.g. determining lung capacity.

Preferably, the aerosol detection unit 12 and the deposition fraction measuring unit 14 are capable of monitoring the air composition of the environment. As shown in Fig. 4A, an air (indicated by the dashed arrow) can enter the device 10C via the first gas opening 22 and reach the aerosol detection unit 12. Upon detection of the incoming air, the deposition fraction measurement unit 14 can measure the air composition, e.g. concentration of particles of different sizes, e.g. diameters.

Such a monitoring of air composition may also be performed by an external monitoring unit 28 shown in Fig. 4A', wherein the external monitoring unit 28 is connected to the device 10C' according to a further embodiment via a wireless link 30. The external monitoring unit 28 may be an air quality detection device, in particular a detector for measuring the size and/or the concentration of particulate matter.

In both cases shown in Figs. 4A, 4A', the monitoring of air composition may be performed on a continuous basis or alternatively activated according to a predefined time frame/schedule, which may be the same schedule for lung data assessment to be performed by the patient. This means that the air composition monitoring may be activated autonomously, i.e. without user/patient inputs for predefined time intervals; or alternatively by the patient, e.g. via patient input.

Based on the air composition monitoring, a time point and/or location for determining the respiratory feature can be defined by the guiding unit 26. In particular, the guiding unit 26 may signal the user to perform lung status assessment when a "bolus" composition, i.e. characterized by a predefined aerosol particle distribution and concentration, is monitored by the device 10C, 10C' or the external monitoring unit 28. The bolus composition is preferably suitable for lung status assessment and can be monitored e.g. in the house, depending on the activities and location inside the house and/or locally (i.e. at a specific location and time). Preferably, the air composition is continuously monitored so that, based on the instantaneously measured particles/size distribution, the guiding unit 26 can determine the optimal moment(s) to signal the patient to perform a new measurement.

In a preferable embodiment, the external monitoring unit 28 may be positioned closely distanced from the device. In this way, the lung assessment can be performed/ prepared/predicted by the device 10C' in close vicinity of the external monitoring unit 28. In particular, the air composition monitored by the external monitoring unit 28 is essentially the same as that present at the device 10C'. The optimal time point and/or location can thus be defined with higher reliability. Further, the result of lung assessment can be reliably predicted based on the monitored air composition.

Alternatively, the optimal time for performing a lung status assessment can be defined based on pre-set time intervals/schedules or based on patient inputs.

Fig. 4B shows schematically the device 10C, 10C' shown in Figs. 4A, 4A' operated during an inhalation phase of a subject 32.

As shown in Fig. 4B, the subject 32 being a patient generates a breathing gas by inhaling through the device 10C, 10C'. In this way, an air from the surrounding enters the device 10C, 10C' via the second gas opening 24 and reaches the aerosol detection unit 12 before subsequently leaving the device 10C, 10C' via the first gas opening 22 and finally entering the respiratory tract 34 of the patient 32. In Fig. 4B, the respiratory tract 34 is only schematically shown and does not reflect the real form or position of the different airways of the respiratory tract in reality. The aerosol detection unit 12 detects an aerosol content in the breathing gas, so that the deposition fraction measurement unit 14 can measure the particle size distribution and/or concentration of the detected aerosol. This can be done during the entire inhalation phase of the patient 32.

Fig. 4C shows schematically the device 10C, 10C' operated during an exhalation phase of the subject 32.

As shown in Fig. 4C, a breathing gas is generated by the respiratory tract 34 of the patient 32, by exhaling into the device 10C, 10C' via the first gas opening 22. The exhaled breathing gas reaches the aerosol detection unit 12, where an aerosol can be detected from the exhaled gas. Upon detection of the aerosol, the deposition fraction measurement unit 14 can measure the particle size distribution and/or concentration. This can be done during the entire exhalation phase of the patient 32.

Based on the particle size distribution measured during the inhalation phase and the subsequent exhalation phase on the subject 32, the fraction of the aerosol that is retained or deposited in the respiratory tract 34 after the breath cycle can be derived leading to the deposition fraction for the aerosol detected in the inhalation phase and the subsequent exhalation phase of the breath cycle. This is performed by the deposition fraction measurement unit 14.

Based on the measured deposition fraction, the respiratory feature determination unit 16 can determine a respiratory feature of the patient 32 by relating the measured deposition fraction to a plurality of predetermined position fractions each corresponding to a different airway geometry of the human respiratory tract.

Preferably, the relating step is performed by presenting the measured deposition fraction as a function (e.g. summation) of the plurality of predetermined deposition fractions or the sub functions of the predetermined deposition fraction, wherein the predetermined deposition fractions or their subfunctions are each multiplied by a corresponding weighting factor (e.g. polynomials, exponential functions and/or logarithm functions). Further preferably, the respiratory feature is derived from the plurality of weighting factors in the function representing the measured deposition fraction based on the predetermined deposition fraction. For instance, the weighting factor corresponding to one or more specific airway geometry of the human respiratory tract is compared with a predefined threshold which is characteristics for a respiratory feature. Alternatively or additionally, the ratio between at least two weighting factors each corresponding to a specific airway geometry of the human respiratory tract is determined and compared with a predefined ratio which is characteristics for a respiratory feature.

Besides representing the measured deposition fraction as a function of the predetermined deposition fractions, the respiratory feature determination unit may compare the measured deposition fraction with one or more of the predetermined deposition fractions regarding the form and/or value of the measured curve. Alternatively or additionally, the respiratory feature determination unit 16 may compute a correlation function between the measured deposition fractions and one or more of the predetermined deposition fractions.

Fig. 5A shows schematically a device 10D according to a further embodiment.

The device 10D is similar to the device 10D shown in Fig. 3 except that the device 10D further comprises a first and a second gas opening 22, 24 as well as a guiding unit 26. In this way, the embodiment shown in Fig. 5A is similar to that shown in Fig. 4A', except that the monitoring unit 20 of the device 10D shown in Fig. 5A is integrated into the device 10D together with the assessment section 18, whereas the monitoring unit 28 in Fig. 4A' is an external unit (e.g. a smart air cleaner, air conditioner or the like).

The afore-mentioned embodiments of the device 10A, 10B, 10C, 10C', 10D are preferably a portable/wearable device, in particular a wearable lung assessment device. In case of the device 10C' shown in Fig. 4A', the device 10C' preferably functions as a "master", to which monitoring data can be sent from the external monitoring unit 28, which preferably functions as a "slave".

Upon monitoring the air composition of the environment by the integrated monitoring unit 20, a guiding unit 26 of the device 10D defines a time and/or location for assessing the respiratory tract 34 of a patient 32, similarly to the case using the device 10C, 10C' as described above. As shown in Fig. 5B, the device 10D is operated during an inhalation phase of the patient 32, similarly to the case described in conjunction with Fig. 4B.

As show in Fig. 5C, the device 10D is operated during an exhalation phase of the patient 32, similarly to the case described above in conjunction with Fig. 4C. Therefore, the device 10D is capable of determining a respiratory feature based on detecting an aerosol in the breathing gas of the patient 32 and measuring the deposition fraction of the detected aerosol. The respiratory feature can be determined by relating the measured deposition fraction to a plurality of predefined deposition fractions each corresponding to a different airway geometry, e.g. parts/sections marked of the human respiratory tract.

Preferably, guiding unit 26 of the embodiment mentioned above comprises a user interface (e.g. display, audio signal element, vibration signal element, light signal element, ... etc.) for signaling the patient to perform lung assessment at a specific time, preferably an optimal time that is defined by the guiding unit 26 as mentioned above. The user interface may be provided to guide the patient 32 to a specific location (e.g. the living room, the kitchen, the sleeping room, the balcony, etc.) where the lung assessment is to be performed. Further, the guiding unit 26 may be configured to first guide the patient 32 to the kitchen where cooking is ongoing and to signal the patient 32 to take a deep inhale. Subsequently, the guiding unit 26 may guide the patient 32 to enter the nearby living room and signal the patient 32 to exhale. In addition, the user interface of the guiding unit 26 may additionally signal the patient 32 to perform one or more breath cycles in the living room.

Fig. 6 shows schematically an example of deposition fraction plotted against aerosol size distribution corresponding to three different indoor events indicated in the inset.

As shown in the inset graph, the concentration of "PM 2.5" particulate matter has been measured for three events E1, E2 and E3, wherein the events E1 and E3 are two different cooking events while the event E2 corresponds to the concentration at background level. The main graph in Fig. 6 shows the aerosol size distribution M1, M2 and M3 measured for the three events F1 to E3, respectively. The size distribution is defined as concentration of the detected aerosol particles depending on the particle size (here in units of μm), wherein the concentration values measured at the specific particle sizes are scaled to the total concentration of all detected aerosol particles.

In the following, a general method for calculating respiratory parameters from aerosol size distributions according to the present invention is described which can be used by any of the embodiments according to the present invention. In particular, the general method is used to calculate the lung depth from size distributions measured for the inhaled and the exhaled aerosol.

Based on the inhaled and exhaled aerosol concentrations ($C_i$, $C_e$) measured for at least two specific particle diameters $d$, the deposition fraction ($DF$) can be defined according to equation (1)

$$DF(d) = 1 - \frac{C_e(d)}{C_i(d)} \qquad (1)$$

The deposition fraction $DF$ as function of aerosol diameter for specific airway geometries (e.g. nasal cavity, trachea, alveoli, etc.) can be predetermined. In practice, the inhaled air volume will travel many different segments of the airways and lungs, so the measured deposition fraction from a single breathing cycle will represent a weighted average of the deposition fractions corresponding to different airway geometries/types (i.e. a summation of the predetermined deposition fractions each multiplied by a weighting factor corresponding to a specific airway geometry of the respiratory tract). This means that the total deposition fraction $DF_{Total}$ is a weighted summation of the predetermined deposition fractions, e.g. the tracheobronchial (upper airway) and pulmonary (smaller/lower airway) deposition fraction curves.

The weighting factors are each determined by the fraction of inhaled volume as contained within the corresponding types of airway. For instance, since COPD patients typically have a more shallow breathing, a smaller percentage of the inhaled volume will reach the smallest airways and a larger percentage will not reach further than the upper airways. This means that the percentage of inhaled volume that reaches the smaller airways is smaller than that of inhaled volume reaching the upper airways. Therefore, compared to the total deposition fraction $DF_{Total}$ (d) of healthy persons, the total deposition fraction curve $DF_{Total}$ (d) of COPD patients will be more predominated by the tracheobronchial deposition fraction curve. Since the deposition fraction $DF(d)$ can be predetermined for each airway type/geometry, the weighting factors corresponding to specific airway geometries can be calculated if $DF_{Total}$ (d) is measured at a sufficient number of aerosol sizes, e.g. diameter values. Respiratory features like depth of breathing and lung capacity can be inferred from the calculated weighting factors. The number of aerosol sizes may vary from at least 2 up to 100 size classes (bins), starting from ultrafine particle size range (i.e. <200nm) to large particle sizes that represent large bioaerosols like pollens, e.g. 100μm-200 μm.

Without loss of generality, the above approach can be extended and refined to include many different airway types encountered in the human or animal respiratory tract.

Fig. 7 shows schematically a procedure flow scheme for lung capacity determination using one of the above embodiments, e.g. shown in Figs. 2-5C.

The procedure flow scheme includes three loops: a "sample input control loop" (loop I), a "measurement loop" (loop II) and a "patient control loop" (loop III). During loop I, air is sensed by an aerosol sensor 36 in order to detect an aerosol in the environment of the patient. In loop II, the air of the same environment can be sensed by another aerosol sensor 38 upon inhalation of air by the patient. This means that the inhalation by the patient generates a breathing gas which first reaches the aerosol sensor 38 before reaching the patient. The aerosol sensor 38 detects an aerosol contained in the breathing gas. Subsequently, when the patient exhales the breathing gas, the exhaled gas is sensed by the aerosol sensor 38.

The amount and/or size distribution of the detected aerosol can be measured by the aerosol sensors 36, 38 in the respective loop and, in the case of loop II, during the inhalation and exhalation of the patient. Preferably, the measurement result of the first aerosol sensor 36 can be provided to the second aerosol sensor 38, e.g. for monitoring/detecting possible changes of air composition during the time interval between performing of both loops I, II. Alternatively or additionally, the measurement result of the first aerosol sensor 36 can be used to define an optimal time point of performing lung capacity assessment using the second aerosol sensor 38.

During loop II, the aerosol sensor 38 is used to measure the deposition fraction of the detected aerosol based on the amount, volume and/or concentration of the inhaled aerosol particles and that of the exhaled aerosol particles. The lung capacity is then determined by relating the measured deposition fraction to a plurality of predetermined deposition fractions each corresponding to a different airway geometry of the human respiratory tract, preferably deposition fractions predetermined for the patient himself.

During loop III, the breathing gas generated by the patient is sensed by an respiratory assistance apparatus, e.g. breathing rate sensors 40 which output a breathing rate of the patient, for instance to a breathing pattern input unit 42. Preferably, the breathing rate sensors 40 may accept one or more breathing pattern inputs from the breathing pattern input unit 42 to assist the patient's breathing. This can be done using a positive airway pressure (PAP) device, in particular a continuous positive airway pressure (CPAP) device, which generates a positive airway pressure, in particular a continuous pressure, that is applied to the patient's respiratory tract.

In a preferable embodiment, the measurement of lung status in loop II may be used to optimize the breathing assistance of a CPAP device, e.g. in a CPAP therapy, while minimizing the pressure that is necessary to achieve the desired treatment effect. For instance, respiratory features, e.g. lung capacity, are monitored by the device according to any of the afore-mentioned embodiments, such a device 10A-D. A patient-specific relationship can be established that links the CPAP pressure to the determined lung capacity. In particular, a desired lung capacity can be pre-set, wherein the measured lung capacity is compared to the pre-set lung capacity, e.g. one or more values of the parameters shown in Fig. 1A. The level of CPAP pressure applied by the CPAP device in the control loop II can be reduced stepwise, wherein after each pressure reduction step the lung capacity is measured again and compared to the pre-set lung capacity. This procedure can be carried out until the measured lung capacity differs from the pre-set lung capacity, e.g. by an amount that succeeds a pre-defined threshold. In this way, one can determine the minimum CPAP pressure level that can be used to assist the patient's breathing while still achieving the desired lung capacity, e.g. during a lung treatment.

Preferably, a flow sensor 44 may be applied, e.g. connected between the patient and the aerosol sensor 38 and/or to the breathing rate sensors 40. The flow sensor 44 is configured to measure the flow, in particular the volume and/or speed of breathing gas generated by the patient. The measured flow value can be provided to the aerosol sensor 38, e.g. for taking the flow value into account when determining the lung capacity based on the detected aerosol. Alternatively or additionally, the measured flow value can be provided to the breathing rate sensors 40, e.g. for optimizing the breathing pattern input/the CPAP pressure applied to the patient.

Additionally or alternatively, the aerosol sensor 38 of the loop II is contained in a device for determining respiratory features, in particular one of the above embodiments 10A-D shown in Figs. 2-5C. In this way, the device 10A-D and the respiratory assistance apparatus, e.g. the breathing rate sensors 40 and preferably the breathing pattern input unit 42, further preferably a CPAP device, form a system for determining a respiratory feature of a subject, in particular a living being, based on a breathing gas.

Figs. 8-9 show schematically another procedure for lung capacity assessment.

As shown in Fig. 8A, a sensor 46 that comprises an aerosol sensor, and preferably a flow sensor, is operated during an inhalation phase (indicated by dashed arrows) followed by an exhalation phase (indicated by solid arrows). During the inhalation phase, air is inhaled by the patient through the sensor 46 which detects an aerosol and measures the amount, volume, concentration and/or size distribution of the detected inhaled aerosol (in the sense that the same aerosol that is detected by the sensor 46 is subsequently inhaled by the patient). During the exhalation phase, air is exhaled by the patient into the sensor 46 which again detects an aerosol and measures the amount, volume, concentration and/or size distribution of the detected exhaled aerosol. The ratio of the measured value (e.g. amount, volume, concentration and/or size distribution) between the inhalation phase and the exhalation phase can therefore be calculated and/or monitored, which possibly results in a changing ratio. The sensor 46 is for instance a part of the devices 10A-D shown in Figs. 1-5C.

The inhalation and/or exhalation may be performed in a selected area in the house or the like where the air composition has been previously monitored. In particular, the breath cycle may be performed at the moment when the monitored air contains a high concentration of aerosol particles.

Fig. 8B shows the number of particles as a function of particle size for the inhalation phase (IP, shown as dashed curve). Further, the number of particles depending on particle size is also plotted for a COPD instable patient ($C_i$), a COPD stable patient ($C_s$), and a person having no COPD (N) each during an exhalation phase. Among the different types of persons (i.e. COPD-instable, COPD-stable and no COPD), the COPD-instable patient is expected to have the lowest lung capacity and shallowest breath. The COPD-stable patient is expected to have a higher lung capacity and shallower breath. The person without COPD is expected to have the highest lung capacity and the deepest breath. As a result, the particle concentration of the deposited breathing gas is expected to also be the lowest for the COPD-instable patient, followed by the COPD-stable patient and then the person having no COPD. Consequently, the particle concentration of the exhaled particle concentration is the highest for the COPD-instable patient, followed by the COPD-stable patient and then the person having no COPD. This is shown in the diagram of Fig. 8B. It can be seen that the higher the measured particle concentration (i.e. number of particles per volume), the lower the deposited fraction of particles and consequently the lower the lung capacity, and vice versa.

The curves IP, $C_i$, $C_s$ and N in Fig. 8B are not to be understood as reflecting real measurment results, but based on hypothetical estimations involving technical and clinical insights. Figs. 8A-8B therefore show a (hypothetical) evolution of the size distribution changes.

Similar measurements are performed as shown schematically in Fig. 9A. In particular, a first inhalation phase is performed by the patient in which air is inhaled by the patient through the sensor 46, as indicated by the dashed arrows. Subsequently, one or more breath cycles each consisting of an inhalation phase followed by an exhalation phase is performed by the patient, in which air is inhaled through an air purifier 48 and exhaled to the sensor 46, as indicated by the solid arrows. The sensor 46 detects an aerosol and measures the amount, volume, concentration and/or size distribution of the detected inhaled and exhaled aerosol. Analogously to Fig. 8A, the ratio of the measured value (e.g. amount, volume, concentration and/or size distribution) between the inhalation phase and the exhalation phase can be calculated and/or monitored, which possibly results in a changing ratio.

By using the air purifier 48, it is achieved that the air inhaled and exhaled during the subsequent breath cycle(s) following the first inhalation phase is purified that e.g. contains a lower concentration of aerosol particles than the air inhaled during the first inhalation phase. The air purifier 48 can be physically connected to the sensor 46 so that they are located in an area with the same air composition. Alternatively or additionally, the air purification can be performed in e.g. a part of the house where the air has been already purified previously.

Fig. 9B shows the number of particles as a function of particle size for a COPD instable patient (C_i), a COPD stable patient (C_s), and a person having no COPD (N) each during an exhalation phase, similarly to the case shown in Fig.8B. The measurement of the size distribution of Fig. 9B can be performed during purification of air, e.g. once after every purification period. Figs. 9A-9B therefore show the effect of clearance of particles from the dirty air (as inhaled in the 1st inhalation phase).

By purifying the air, the amount/concentration of particles present in the air retained in the lung(s) is gradually lowered, and the data is used to do the various assessments of the lung status (e.g. lung capacity, pulmonary volume, depth of inhalation, exhaled capability of the patient, etc.). In particular, the above approach is tuned to assess the functional residual capacity (FRC) of the patient, which is a highly relevant parameter in the assessment of respiratory status in diseases such as COPD, as shown in Fig. 1. If the patient inhales clean air (i.e. air which contains either no or insignificant amount of particles), the deposition fraction of aerosol in the exhaled breathing gas can serve as an indicator of the aerosols retained in the lung. This relates to the residual capacity of the lungs. This information may be extracted when the patient performs inhalation of clean air only and subsequently exhalation to the aerosol sensor. The measured aerosol concentration and preferably also the size range then will be used for estimating the residual capacity. A temporal analysis of the aerosol concentration of the exhaled gas can be needed, preferably until the aerosol concentration reaches to a value/distribution of a 'clean' state, in which the particle concentration is close to zero or lower than a threshold.

The present invention provides ambulatory pulmonary function tests which are reliable and capable of supporting short-term assessment in the management of respiratory diseases, such as COPD, asthma, and lung emphysema. Such tests can be used for a variety of applications, such as continuous monitoring and personalization of treatment (e.g. time and dose of medication) and early detection of infections in respiratory conditions to provide an early warning of exacerbations and enable the patient or doctor to adjust medication in a timely fashion. In addition, the test may provide patients feedback regarding their inhalation technique and, as a result, it may be used to optimize inhalation of respiratory drugs.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Device for determining a respiratory feature of a subject based on a breathing gas generated by the subject when inhaling and exhaling, comprising:

- an aerosol detection unit (12) for detecting an aerosol contained in the breathing gas;
- a deposition fraction measurement unit (14) for measuring a deposition fraction for the detected aerosol, wherein the deposition fraction indicates the fraction of aerosol deposited inside the subject over the total amount of the inhaled aerosol, wherein the deposition fraction measurement unit (14) is configured to measure a particle concentration for a plurality of particle sizes of the detected aerosol, and wherein the deposition fraction comprises a plurality of values each corresponding to a particle size of the plurality of particle sizes; and
- a respiratory feature determination unit (16) for determining the respiratory feature by relating the measured deposition fraction to a plurality of predetermined deposition fractions each corresponding to a different airway geometry of a respiratory tract, wherein each airway geometry

2. The device according to claim 1, wherein the deposition fraction measurement unit (14) is configured to derive a particle size distribution of the detected aerosol and/or to derive a total deposition fraction being the sum of deposition fractions for the plurality of particle sizes of the detected aerosol.

3. The device according to claim 2, wherein the deposition fraction measurement unit (14) is configured to derive a ratio between the size distribution of aerosol detected during an inhalation phase and that of aerosol detected during an exhalation phase.

4. The device according to claim 1, wherein at least one of the plurality of predetermined deposition fractions corresponds to an upper airway, a lower airway, a tracheobronchial airway and/or a pulmonary airway.

5. The device according to claim 1, wherein at least one of the plurality of predetermined deposition fractions corresponds to an airway geometry of the subject.

6. The device according to claim 1, wherein the respiratory feature determination unit (16) is configured to represent the measured deposition fraction as a function of the plurality of predetermined deposition fractions.

7. The device according to claim 6, wherein the function comprises summation of the plurality of predetermined deposition fractions or their subfunctions, wherein the predetermined deposition fractions or their subfunctions are each multiplied by a corresponding one of a plurality of weighting factors.

8. The device according to claim 7, wherein the respiratory feature determination unit (16) is configured to derive the respiratory feature from the plurality of weighting factors.

9. The device according to claim 1, further comprising a monitoring unit (20) for monitoring an air composition.

10. The device according to claim 1, wherein the device is a portable or wearable device, and/or connectable to an external monitoring unit (28) for monitoring an air composition.

11. The device according to claim 1, further comprising a guiding unit (26) for setting a time point and/or location for determining the respiratory feature.

12. System for determining a respiratory feature of a subject based on a breathing gas generated by the subject when inhaling and/or exhaling, comprising:

- a respiratory assistance apparatus (40, 42) for assisting the respiration of the subject; and
- a device (10A-D) as claimed in claim 1 for determining a respiratory feature of the subject assisted by the respiratory assistance apparatus.

**Patentansprüche**

1. Gerät zum Ermitteln eines Atmungsmerkmals einer Person anhand der von der Person beim Ein- und Ausatmen erzeugten Atemluft, das Folgendes umfasst:

- eine Aerosol-Erfassungseinheit (12) zum Erfassen des in der Atemluft enthaltenen Aerosols;
- eine Einheit zum Messen des Ablagerungsanteils (14) zum Messen des Ablagerungsanteils des erfassten Aerosols, wobei der Ablagerungsanteil den Anteil des Aerosols angibt, der sich im Verhältnis zur Gesamtmenge des eingeatmeten Aerosols im Inneren der Person ablagert, wobei die Einheit zum Messen des Ablagerungsanteils (14) für mehrere Partikelgrößen des erfassten Aerosols die Partikelkonzentration misst, und wobei der Ablagerungsanteil mehrere Werte umfasst, die jeweils einer Partikelgröße der mehreren Partikelgrößen entsprechen; und
- Einheit zum Ermitteln eines Atmungsmerkmals (16) zum Ermitteln des Atmungsmerkmals, indem der gemessene Ablagerungsanteil mit mehreren vorab festgelegten Ablagerungsanteilen in Beziehung gesetzt wird, die jeweils einer unterschiedlichen Luftweggeometrie der Atemwege entsprechen, wobei die einzelnen Atemwegsgeometrien jeweils einem anderen Teil oder Abschnitt des Atemwegs entsprechen.

2. Das Gerät gemäß Anspruch 1, wobei die Einheit zum Messen des Ablagerungsanteils (14) die Partikelgrößenverteilung des erfassten Aerosols und/oder den Gesamtablagerungsanteil ableitet, bei dem es sich um die Summe der Ablagerungsanteile für die mehreren Partikelgrößen des erfassten Aerosols handelt.

3. Das Gerät gemäß Anspruch 2, wobei die Einheit zum Messen des Ablagerungsanteils (14) das Verhältnis zwischen der Größenverteilung des während einer Einatmungsphase erfassten Aerosols und derjenigen des während einer Ausatmungsphase erfassten Aerosols ableitet.

**4.** Das Gerät gemäß Anspruch 1, wobei mindestens einer der mehreren vorab festgelegten Ablagerungsanteile einem oberen Luftweg, einem unteren Luftweg, einem tracheobronchialen Luftweg und/oder einem pulmonalen Luftweg entspricht.

**5.** Das Gerät gemäß Anspruch 1, wobei mindestens einer der mehreren vorab festgelegten Ablagerungsanteile der Atemwegsgeometrie des Patienten entspricht.

**6.** Das Gerät gemäß Anspruch 1, wobei die Einheit zum Ermitteln von Atmungsmerkmalen (16) den gemessenen Ablagerungsanteil als eine Funktion der mehreren vorab festgelegten Ablagerungsanteile darstellt.

**7.** Das Gerät gemäß Anspruch 6, wobei die Funktion das Summieren der mehreren vorab festgelegten Ablagerungsanteile oder deren Unterfunktionen umfasst, wobei die vorab festgelegten Ablagerungsanteile oder deren Unterfunktionen jeweils entsprechend mit einem von mehreren Gewichtungsfaktoren multipliziert werden.

**8.** Das Gerät gemäß Anspruch 7, wobei die Einheit zum Ermitteln des Atmungsmerkmals (16) das Atmungsmerkmal aus den mehreren Gewichtungsfaktoren ableitet.

**9.** Das Gerät gemäß Anspruch 1, das zudem eine Überwachungseinheit (20) zum Überwachen der Luftzusammensetzung umfasst.

**10.** Das Gerät gemäß Anspruch 1, wobei es sich bei dem Gerät um ein mobiles oder tragbares Gerät und/oder um ein Gerät handelt, das mit einer externen Überwachungseinheit (28) zum Überwachen der Luftzusammensetzung verbunden werden kann.

**11.** Das Gerät gemäß Anspruch 1, das zudem eine Führungseinheit (26) zum Einstellen des Zeitpunkts und/oder der Position für das Ermitteln des Atmungsmerkmals umfasst.

**12.** System zum Ermitteln eines Atmungsmerkmals einer Person anhand der von der Person beim Ein- und/oder Ausatmen erzeugten Atemluft, das Folgendes umfasst:

- ein Gerät zur Unterstützung der Atmung (40, 42) zum Unterstützen der Atmung der Person; und
- ein Gerät (10A-D) gemäß Anspruch 1 zum Ermitteln eines Atmungsmerkmals der Person, das vom Gerät zur Unterstützung der Atmung unterstützt wird.

**Revendications**

**1.** Dispositif de détermination d'une caractéristique respiratoire d'un sujet en fonction d'un gaz respiratoire généré par le sujet lors de l'inspiration et de l'expiration, comprenant:

- une unité de détection d'aérosol (12) permettant de détecter un aérosol contenu dans le gaz respiratoire;
- une unité de mesure de fraction de dépôt (14) permettant de mesurer une fraction de dépôt pour l'aérosol détecté, dans lequel la fraction de dépôt indique la fraction d'aérosol déposée à l'intérieur du sujet rapporté à la quantité totale d'aérosol inhalé, dans lequel l'unité de mesure de fraction de dépôt (14) est conçue pour mesurer une concentration de particules pour une pluralité de tailles de particules de l'aérosol détecté, et dans lequel la fraction de dépôt comprend une pluralité de valeurs correspondant respectivement à une taille de particule de la pluralité de tailles de particules; et
- une unité de détermination de caractéristique respiratoire (16) permettant de déterminer la caractéristique respiratoire par mise en relation de la fraction de dépôt mesurée avec une pluralité de fractions de dépôt prédéterminées correspondant respectivement à une géométrie différente des voies aériennes des voies respiratoires, dans lequel chaque géométrie des voies aériennes correspond à une partie ou une section différente desdites voies respiratoires.

**2.** Dispositif selon la revendication 1, dans lequel l'unité de mesure de fraction de dépôt (14) est conçue pour dériver une distribution de tailles de particules de l'aérosol détecté et/ou pour dériver une fraction de dépôt totale étant la somme des fractions de dépôt pour la pluralité de tailles de particules de l'aérosol détecté.

**3.** Dispositif selon la revendication 2, dans lequel l'unité de mesure de fraction de dépôt (14) est conçue pour dériver un rapport entre la distribution de tailles de l'aérosol détecté pendant une phase d'inspiration et celle de l'aérosol détecté pendant une phase d'expiration.

**4.** Dispositif selon la revendication 1, dans lequel l'au moins une fraction de la pluralité de fractions de dépôt prédéterminées correspond à des voies aériennes supérieures, des voies aériennes inférieures, des voies aériennes trachéobronchiques et/ou des voies aériennes pulmonaires.

**5.** Dispositif selon la revendication 1, dans lequel l'au moins une fraction de la pluralité de fractions de dépôt prédéterminées correspond à une géométrie des voies aériennes du sujet.

6. Dispositif selon la revendication 1, dans lequel l'unité de détermination de caractéristique respiratoire (16) est conçue pour représenter la fraction de dépôt mesurée en fonction de la pluralité de fractions de dépôt prédéterminées.

7. Dispositif selon la revendication 6, dans lequel la fonction comprend la sommation de la pluralité de fractions de dépôt prédéterminées ou de leurs sous-fonctions, dans lequel les fractions de dépôt prédéterminées ou leurs sous-fonctions sont respectivement multipliées par l'un correspondant d'une pluralité de facteurs de pondération.

8. Dispositif selon la revendication 7, dans lequel l'unité de détermination de caractéristique respiratoire (16) est conçue pour dériver la caractéristique respiratoire de la pluralité de facteurs de pondération.

9. Dispositif selon la revendication 1, comprenant en outre une unité de commande (20) permettant de commander une composition de l'air.

10. Dispositif selon la revendication 1, dans lequel le dispositif est un dispositif portatif ou portable, et/ou pouvant être connecté à une unité de surveillance (28) externe permettant de surveiller une composition de l'air.

11. Dispositif selon la revendication 1, comprenant en outre une unité de guidage (26) permettant de définir un moment et/ou un emplacement pour déterminer la caractéristique respiratoire.

12. Système de détermination d'une caractéristique respiratoire d'un sujet en fonction d'un gaz respiratoire généré par le sujet lors de l'inspiration et/ou de l'expiration, comprenant :

   - un appareil d'aide à la respiration (40, 42) permettant d'assister la respiration du sujet ; et
   - un dispositif (10A-D) selon la revendication 1 permettant de déterminer une caractéristique respiratoire du sujet aidé par l'appareil d'aide respiratoire.

FIG.1A                    FIG.1B

FIG.2

FIG.3

FIG.4A

FIG.4A'

FIG.4B

FIG.4C

FIG.5A

12

22

14

16

20

24 10D

26

18

FIG.5B

32

34

22

12

14

16

20

24 10D

26

18

FIG.5C

32

34

22

12

14

16

20

24 10D

26

18

FIG.6

FIG.7

FIG.8

FIG.9

**EP 3 359 035 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014076250 A1
- US 2005066968 A1
- US 20050045175 A1